# EUROPÄISCHE PATENTANMELDUNG

(11) **EP 4 454 743 A1**
(43) Veröffentlichungstag der Anmeldung: **30.10.2024**
(21) Anmeldenummer: 23169676.6
(22) Anmeldetag: 25.04.2023
(51) Int. Cl.: B01F 29/15, B01F 33/25, A61K 9/50

(54) **NEUE VERWENDUNGEN VON FLUORCARBONEN**

(71) Anmelder: Midas Pharma GmbH, 55218 Ingelheim (DE)
(72) Erfinder: Primaveßy, Daniel Andreas Addi, 55437 Appenheim (DE); Saaler-Reinhardt, Sigrid, 55291 Saulheim (DE)

(57) **Zusammenfassung**

Die vorliegende Erfindung betrifft die Verwendung von pharmazeutisch akzeptablen, flüssigen Stromstörern, Fluorcarbonen 2, zur Erzeugung von Turbulenzen in flüssigen und halbfesten pharmazeutischen Zubereitungen 1 und damit deren effizienterem Durchmischen vor dem Gebrauch.

## Beschreibung

Arzneimittel zur Injektion sind häufig Suspensionen, Emulsionen, Lösungen mit scherverdünnenden Eigenschaften oder Pulver und Lyophilisate zur Rekonstitution.

Um eine sichere Injektion in den menschlichen oder tierischen Körper zu ermöglichen, muss vor der Anwendung eine homogene Verteilung des Wirkstoffs in der pharmazeutischen Zubereitung sichergestellt werden und die Viskosität der Zubereitung niedrig genug sein.

Suspensionen müssen in der Regel direkt vor der Applikation erneut vermischt werden, da sie zur Aggregierung, Agglomeration oder Sedimentation neigen. Bei längerer Lagerung können sich Sedimente sogar verfestigen und kompakte, feste Ablagerungen ausbilden, die sich nur schwer redispergieren lassen.

Emulsionen weisen wegen des geringeren Dichteunterschieds zwischen zwei Flüssigkeiten eine im Vergleich zu Suspensionen weniger ausgeprägte Sedimentationsneigung auf. Allerdings kommt es recht häufig zur Aggregierung und Agglomerisierung, so dass vor der Anwendung ebenfalls ein Durchmischen erforderlich ist.

Lösungen mit scherverdünnendem Effekt neigen dazu, im Ruhezustand Flüssigkristalle zu bilden, was zu einer enormen Erhöhung der Viskosität führt. Um die Viskosität wieder zu reduzieren, ist es notwendig, die Bindungen zwischen den Molekülen durch Vermischen aufzubrechen.

Pulver und Lyophilisate zur Rekonstitution müssen vor der medizinischen Anwendung in einem flüssigen Lösemittel vollständig aufgelöst werden. Je nach den Lösungseigenschaften des Pulvers erfolgt der Auflösungsvorgang sehr langsam, kann aber durch eine zusätzliche Durchmischung stark beschleunigt werden.

In jedem der oben genannten Fälle muss die pharmazeutische Zubereitung vor ihrer Anwendung gemischt werden.

Die gebräuchlichste Methode zum Mischen von flüssigen und halbfesten pharmazeutischen Zubereitungen ist die Erzeugung von Turbulenzen innerhalb der Kammer, welche die pharmazeutische Zubereitung enthält. Für die Erzeugung von Turbulenzen ist das Vorhandensein von zwei Phasen mit ausreichend unterschiedlicher Dichte notwendig. In der Regel werden daher Gasblasen mit einer viel niedrigeren Dichte als die flüssige pharmazeutische Zubereitung oder feste Kugeln mit einer deutlich höheren Dichte als die flüssige pharmazeutische Zubereitung eingesetzt.

Zum Beispiel ist BYDUREON BCise^{®} (AstraZeneca) ein Medikament, das den pharmazeutischen Wirkstoff Exenatid enthält, der in Polymerpartikel formuliert ist, welche wiederum in einer flüssigen Phase aus mittelkettigen Triglyceriden suspendiert sind. Der hohe Dichteunterschied zwischen den Polymerpartikeln und den flüssigen Triglyceriden führt zu einer schnellen Sedimentation. Vor der Anwendung des Medikaments muss der Patient das Sediment durch intensives Schütteln wieder verteilen. Die Redispergierung der Suspension ist nur möglich, weil die Apparatur eine große Gasblase enthält, die als Stromstörer wirkt, d.h. dessen Dichte unterschiedlich von der Dichte der pharmazeutischen Zubereitung ist, so dass die zur Vermischung notwendigen Turbulenzen durch Schütteln erzeugt werden können.

Gasblasen als Stromstörer haben jedoch mehrere Nachteile:
Erstens müssen Gasblasen vor der Injektion aus dem Primärpackmittel entfernt werden. Daraus ergibt sich eine komplexe und fehleranfällige Handhabung der Apparatur bis hin zu einer falschen Dosierung des therapeutischen Medikaments.

Zweitens können Gasblasen eine Oberfläche ausbilden, an der sich hydrophobe oder hydrophile Domänen empfindlicher Wirkstoffe wie Proteine entfalten und damit zum Abbau des Wirkstoffs und zu dessen Unwirksamkeit führen können.

Bei zu starkem Schütteln der Apparatur kann drittens die Gasblase die Bildung eines persistenten Schaums verursachen, der erst nach Stunden wieder verschwindet.

Viertens können Patienten Apparaturen mit einer Gasblase fälschlicherweise als beschädigt oder unbrauchbar betrachten.

Fünftens nehmen Gasblasen viel Platz in der Apparatur ein, der für die Aufnahme der pharmazeutischen Zubereitung verloren geht.

Feste Kugeln weisen ebenfalls mehrere Nachteile auf:
Feste Kugeln als Stromstörer können den Auslass von Spritzen verstopfen und somit die gesamte Apparatur unbrauchbar machen. Darüber hinaus können sie die Bewegung des Spritzenkolbens blockieren, was zur Erhöhung des Totvolumens führt.

Des Weiteren können feste Kugeln hohe Scherkräfte erzeugen, die zur Beschädigung empfindlicher Bestandteile der pharmazeutischen Zubereitung führen können.

Es besteht Bedarf an verbesserten Methoden und Vorrichtungen, die eine effizientere Redispergierung von flüssigen und halbfesten Arzneiformen ermöglichen. Insbesondere besteht Bedarf an vereinfachten Verfahren zum Redispergieren von Sedimenten, Emulsionen und Flüssigkristallen sowie zur vereinfachten Rekonstitution von Pulvern und Lyophilisaten.

Das Ziel der vorliegenden Erfindung ist es, verbesserte Verfahren und Vorrichtungen zum Durchmischen und Redispergieren von flüssigen und halbfesten Arzneiformen bereitzustellen, welche die Nachteile der aus dem Stand der Technik bekannten Verfahren nicht aufweisen.

Die vorliegende Erfindung betrifft die Verwendung von pharmazeutisch akzeptablen, flüssigen Stromstörern anstelle von gasförmigen oder festen Phasen zur Erzeugung von Turbulenzen in flüssigen und halbfesten pharmazeutischen Zubereitungen und damit deren effizienterem Durchmischen vor dem Gebrauch.

Erfindungsgemäße Vorrichtungen umfassen eine Kammer, welche eine pharmazeutische Zubereitung sowie einen flüssigen Stromstörer enthält.

Erfindungsgemäße flüssige Stromstörer müssen zwei wesentliche Eigenschaften aufweisen. Erstens dürfen sie mit der pharmazeutischen Zubereitung nicht mischbar sein. Zweitens müssen Sie eine im Vergleich zur pharmazeutischen Zubereitung ausreichend unterschiedliche Dichte besitzen, so dass durch Krafteintrag Turbulenzen entstehen, die wiederum eine Durchmischung der pharmazeutischen Zubereitung bewirken.

Erfindungsgemäße Stromstörer wie beispielsweise flüssige Fluorcarbone weisen im Vergleich zu herkömmlichen gasförmigen bzw. festen Stromstörern für den pharmazeutischen Gebrauch vorteilhafte Eigenschaften auf.

Im Gegensatz zu Gasblasen müssen erfindungsgemäße flüssige Stromstörer vor der Anwendung nicht entfernt werden, sondern können mitinjiziert werden. Das führt zu einer vereinfachten Handhabung, da beispielsweise der Entgasungsschritt beim Vorhandensein einer Gasblase entfällt. Zudem wird die Patienten-Compliance verbessert und eine Fehldosierung ausgeschlossen.

Zweitens kann sich aufgrund des Fehlens einer Gasblase selbst durch starkes Schütteln der Vorrichtung kein Schaum bilden.

Durch das Fehlen einer Gasblase werden drittens Patienten Medikamente mit einem flüssigen Stromstörer nicht fälschlicherweise als beschädigt und unbrauchbar betrachten.

Im Vergleich zu festen Kugeln können die in dieser Erfindung beschriebenen flüssigen Stromstörer den Auslass von Spritzen nicht behindern und den Kolben nicht blockieren. Darüber hinaus haben sie ein weitaus geringeres Potenzial, zu hohe Scherkräfte zu erzeugen und damit sensible Bestandteile der pharmazeutischen Zubereitung zu zerstören.

Insgesamt sind die erfindungsgemäßen Verwendungen, Verfahren und Vorrichtungen im Vergleich zu den bekannten Lösungen weniger komplex, einfacher zu handhaben, kompatibler und weisen eine verbesserte Compliance auf.

Probleme wie die Aggregierung und Agglomeration von Emulsionen, die Sedimentation und Sedimentverhärtung von Suspensionen, die hohe initiale Viskosität von scherverdünnenden Flüssigkeiten sowie die langsame Auflösung von Pulvern und Lyophilisaten werden damit überwunden.

Die Erfindung wird im Folgenden in mehreren Anwendungsfällen veranschaulicht.

Ein Gegenstand der vorliegenden Erfindung ist die Verwendung von flüssigen Stromstörern anstatt gasförmiger oder fester Phasen zur Durchmischung von flüssigen sowie halbfesten pharmazeutischen Zubereitungen.

Erfindungsgemäße Vorrichtungen umfassen dabei eine Kammer, welche eine pharmazeutische Zubereitung zur therapeutischen Anwendung bei Menschen oder Tieren sowie einen pharmazeutisch akzeptablen, flüssigen Stromstörer enthält.

Pharmazeutische Zubereitungen im Sinne der vorliegenden Erfindung sind Mischungen aus Wirkstoffen und Hilfsstoffen. Die vorliegende Erfindung ist für flüssige sowie halbfeste Zubereitungen relevant, die mindestens eine flüssige Phase aufweisen. Beispiele für flüssige Zubereitungen sind Emulsionen, Suspensionen und Lösungen. Beispiele für halbfeste Zubereitungen sind Cremes, Gele, Pasten und Salben.

Pharmazeutisch akzeptable flüssige Stromstörer im Sinne der vorliegenden Erfindung sind Substanzen, die eine flüssige Phase bilden, welche mit der pharmazeutischen Zubereitung nicht mischbar ist.

Darüber hinaus weisen erfindungsgemäße flüssige Stromstörer eine von der flüssigen Phase der pharmazeutischen Zubereitung signifikant unterschiedliche Dichte auf. Aus diesem Dichteunterschied resultiert ein unterschiedliches Trägheitsmoment, was bei Krafteintrag (z. B. durch Bewegen der Vorrichtung) zur Induktion von Turbulenzen in der Mischkammer führt, die für eine Durchmischung der pharmazeutischen Zubereitung sorgen.

Eine signifikant geringere Dichte kann ein Dichteunterschied des flüssigen Stromstörers um den Faktor 0,8 oder weniger im Vergleich zur Dichte der flüssigen Phase der pharmazeutischen Zubereitung sein. Eine signifikant höhere Dichte bedeutet einen Dichteunterschied des flüssigen Stromstörers um den Faktor 1,2 oder mehr im Vergleich zur Dichte der flüssigen Phase der pharmazeutischen Zubereitung. In bevorzugten Ausführungsformen liegt das Verhältnis der Dichte des flüssigen Stromstörers zur Dichte der flüssigen Phase der pharmazeutischen Zubereitung im Bereich von 1,3 zu 1 und 3,0 zu 1.

Obwohl erfindungsgemäße flüssige Stromstörer aus pharmakologischer Sicht zusammen mit der pharmazeutischen Zubereitung in den Körper des Patienten appliziert werden können, sind sie im Sinne der vorliegenden Erfindung nicht Bestandteil der pharmazeutischen Zubereitung.

Eine Ausführungsform der Erfindung betrifft daher Vorrichtungen zum Durchmischen von flüssigen oder halbfesten Arzneiformen umfassend eine Kammer, enthaltend
a) eine pharmazeutische Zubereitung umfassend einen oder mehrere pharmazeutische Wirkstoffe sowie eine flüssige Phase, und
b) einen pharmazeutisch akzeptablen, mit der pharmazeutischen Zubereitung nicht mischbaren, flüssigen Stromstörer,
wobei das Verhältnis der Dichte des flüssigen Stromstörers zur Dichte der flüssigen Phase der pharmazeutischen Zubereitung im Bereich von 1,3 zu 1 und 3,0 zu 1 liegt, so dass eine Durchmischung der pharmazeutischen Zubereitung ermöglicht wird.

Bevorzugte pharmazeutisch akzeptable flüssige Stromstörer sind flüssige Fluorcarbone (FCs), also fluorierte Derivate von Kohlenwasserstoffen.

Gegenstand der vorliegenden Erfindung ist daher die Verwendung von einem oder mehreren flüssigen Fluorcarbonen als Stromstörer in flüssigen oder halbfesten pharmazeutischen Zubereitungen, die einen pharmazeutischen Wirkstoff und eine flüssige Phase umfassen.

Weiter sind Verfahren zum Vermischen von flüssigen oder halbfesten pharmazeutischen Zubereitungen mittels einem oder mehrerer flüssiger Fluorcarbone in flüssigen oder halbfesten pharmazeutischen Zubereitungen Gegenstand der vorliegenden Erfindung.

Da die meisten Fluorcarbone gleichermaßen hydrophob und oleo- bzw. lipophob sind, gehen sie nur sehr geringe physikalische Wechselwirkungen mit der pharmazeutischen Zubereitung ein und sind daher mit ihr nicht mischbar.

Fluorcarbone besitzen Dichten von etwa 1,5 bis 2,0 g/ml. Im Vergleich zu flüssigen wässrigen oder öligen Phasen, deren Dichte in der Regel zwischen von 0,8 bis 1,0 g/ml liegt, weisen Fluorcarbone damit weitaus höhere Dichten auf, so dass durch Krafteintrag Verwirbelungen in der pharmazeutischen Zubereitung induziert werden und dadurch ein Mischeffekt bewirkt wird.

Da die meisten Fluorcarbone sowohl hydrophob als auch oleophob sind und somit eine ausgeprägte Phasengrenze zur flüssigen Phase der pharmazeutischen Zubereitung ausbilden, kann die Entfaltung und damit der Abbau von empfindlichen Wirkstoffen wie beispielsweise Proteinen nicht provoziert werden. Die erfindungsgemäßen Fluorcarbone eignen sich daher insbesondere für empfindliche Wirkstoffe wie beispielsweise Aminosäuren, Peptide sowie Proteine, insbesondere Oligopeptide.

Zudem sind FCs klare Flüssigkeiten und unterscheiden sich somit auf den ersten Blick nicht von der pharmazeutischen Zubereitung, so dass der Patient eine intakte Vorrichtung nicht irrtümlich als defekt und unbrauchbar ansehen wird.

Da Fluorcarbone chemisch weitgehend inert sind, können sie zusammen mit der pharmazeutischen Zubereitung sicher in den menschlichen oder tierischen Körper eingebracht werden.

Bevorzugte Fluorcarbone im Sinne der vorliegenden Erfindung sind fluorierte Derivate von Kohlenwasserstoffen mit mindestens 2 und höchstens 18 Kohlenstoffatomen, bevorzugt mindestens 3 und höchstens 14 Kohlenstoffatome.

Die erfindungsgemäßen Fluorcarbone können zudem eines oder mehrere Heteroatome wie Wasserstoff-, Stickstoff-, Sauerstoff-, Schwefel-, Chlor-, Brom- oder lodatome enthalten.

Bevorzugt sind perfluorierte Derivate, deren Fluorierungsgrad, d. h. der Anteil von durch Fluoratome substituierten Wasserstoffatomen mindestens 95%, besonders bevorzugt 99% oder mehr beträgt.

Beispiele für erfindungsgemäße flüssige Fluorcarbone umfassen, sind aber nicht beschränkt auf, Perfluordecalin (PFD), Perfluormethyldecalin (PFMD), Perfluor(tert-Butylcyclohexan), Perfluorisopropyldecalin, Perfluorpropan, Perfluorobutan, Perfluorpentan, Perfluorhexan, Perfluorheptan, Perfluoroktan, Perfluornonan, Perfluordekan, Perfluorundekan und Perfluordodekan. Beispiele für erfindungsgemäße flüssige Fluorcarbone mit einem Heteroatom sind Undekafluorpentan, Perfluortripropylamin, Perfluortributylamin, Perfluormethylcyclo-hexylpiperidin, Perfluoroktylbromid, Perfluordecylbromid sowie Perfluordichloroktan.

Erfindungsgemäß bevorzugte flüssige Fluorcarbone sind Perfluordecalin (PFD) sowie Perfluormethyldecalin (PFMD).

Eine Ausführungsform der Erfindung betrifft die Verwendung von flüssigen Fluorcarbonen in Vorrichtungen zur Injektion von flüssigen oder halbfesten pharmazeutischen Zubereitungen, die eine Kammer zur Aufnahme der pharmazeutischen Zubereitung umfassen, welche eine pharmazeutische Zubereitung mit einem oder mehreren pharmazeutischen Wirkstoffen und eine flüssige Phase enthält.

Eine weitere Ausführungsform der Erfindung betrifft Vorrichtungen zur Injektion von flüssigen oder halbfesten pharmazeutischen Zubereitungen, umfassend eine Kammer, enthaltend
a) eine pharmazeutische Zubereitung umfassend einen oder mehrere pharmazeutische Wirkstoffe sowie eine flüssige Phase, und
b) ein flüssiges Fluorcarbon.

Spezielle Beispiele für erfindungsgemäße pharmazeutische Zubereitungen sind Suspensionen, Emulsionen, Flüssigkeiten mit scherverdünnenden Eigenschaften, die Flüssigkristallstrukturen bilden, sowie Pulver und Lyophilisate zur Rekonstitution.

Eine Ausführungsform der vorliegenden Erfindung betrifft die Verwendung von flüssigen Fluorcarbonen zum Vermischen von pharmazeutischen Zubereitungen in Form einer Suspension, in denen eine feste Phase in einer flüssigen Phase dispergiert ist.

Der pharmazeutische Wirkstoff bzw. die pharmazeutischen Wirkstoffe liegen in dieser Ausführungsform in der festen Phase der pharmazeutischen Zubereitung vor, welche einen oder mehrere pharmazeutisch akzeptable Hilfsstoffe enthält. Der pharmazeutisch akzeptable Hilfsstoff kann ein pharmazeutisch akzeptables Polymer sein. Beispiele für pharmazeutisch akzeptable Polymere sind Poly(milchsäure-co-glykolsäure) (PLGA), Polyglykolsäure, Polymilchsäure, Polycaprolacton, Poly(N-isopropylacrylamid), Gelatine, Poly(methylmethacrylate), Lignin sowie Polysaccharide wie Cellulose, Dextran, Stärke, Chitosan, Alginat und Glucomannan.

Bevorzugtes erfindungsgemäßes pharmazeutisch akzeptables Polymer ist Poly(milchsäure-co-glykolsäure) (PLGA).

Die flüssige Phase der pharmazeutischen Zubereitung kann aus wässrigen und/oder nicht-wässrigen Flüssigkeiten bestehen. In bevorzugten Ausführungsformen der vorliegenden Erfindung besteht die flüssige Phase der pharmazeutischen Zubereitung aus nicht-wässrigen Flüssigkeiten, beispielsweise flüssigen Lipiden, insbesondere Triglyceriden.

Ist die Dichte des flüssigen Stromstörers wie im Fall eines Fluorcarbons höher als die Dichte der flüssigen und festen Phasen der Suspension, bildet er eine flüssige Schicht unterhalb der flüssigen bzw. festen Phasen. Kommt es, z. B. während einer Lagerung, zu einer Sedimentation der Suspension, so lagert sich das Sediment an der leicht beweglichen Grenzfläche zwischen den beiden flüssigen Phasen von Suspension bzw. Fluorcarbon ab, so dass eine Ablagerung des Sediments am Boden der Kammer nicht auftreten und damit eine Verhärtung nicht erfolgen kann.

Auf diese Weise können auch sedimentierte Suspensionen mittels Fluorcarbonen auch ohne eine Gasblase leicht redispergiert werden.

Die Erfindung betrifft damit auch Vorrichtungen zum Durchmischen von pharmazeutischen Zubereitungen zur Injektion in Form einer Suspension, umfassend eine Kammer, enthaltend
a) eine pharmazeutische Zubereitung umfassend einen pharmazeutischen Wirkstoff und eine flüssige Phase,
b) eine in der flüssigen Phase dispergierte feste Phase, sowie
c) ein flüssiges Fluorcarbon.

Darüber hinaus sind alle flüssigen Stromstörer zur Durchmischung von Suspensionen geeignet, die mit der pharmazeutischen Zubereitung nicht mischbar sind und deren Dichte signifikant höher ist als die Dichte der flüssigen und der festen Phase der Suspension, so dass eine Sedimentverhärtung vermieden und eine einfache Redispergierung von sedimentierten Suspensionen ermöglicht wird.

Die Erfindung betrifft daher auch die Verwendung von flüssigen Stromstörern zum Durchmischen von pharmazeutischen Zubereitungen in Form einer Suspension mit einer flüssigen Phase und einer festen Phase, die in der flüssigen Phase dispergiert ist, wobei die Dichte des flüssigen Stromstörers signifikant höher ist als die Dichte der flüssigen und der festen Phase der pharmazeutischen Zubereitung.

Zudem betrifft die vorliegende Erfindung Vorrichtungen zum Durchmischen von Suspensionen umfassend eine Kammer, enthaltend
a) eine pharmazeutische Zubereitung, umfassend
   i. eine flüssige Phase,
   ii. eine feste Phase mit einem oder mehreren pharmazeutischen Wirkstoffen, einem oder mehreren pharmazeutisch akzeptablen Hilfsstoffen, die in der flüssigen Phase dispergiert sind, sowie
b) einen pharmazeutisch akzeptablen, mit der pharmazeutischen Zubereitung nicht mischbaren, flüssigen Stromstörer,
wobei die Dichte des flüssigen Stromstörers signifikant höher ist als die Dichte der flüssigen und der festen Phase der pharmazeutischen Zubereitung, so dass eine Durchmischung der pharmazeutischen Zubereitung ermöglicht wird.

In bevorzugten Ausführungsformen ist das Verhältnis der Dichte des flüssigen Stromstörers zur Dichte der festen Phase der Suspension höher als 1,1 zu 1, bevorzugt zwischen 1,2 zu 1 und 2,5 zu 1, besonders bevorzugt im Bereich von 1,3 zu 1 bis 2,0 zu 1.

Eine weitere Ausführungsform der vorliegenden Erfindung betrifft die Verwendung von flüssigen Fluorcarbonen zum Vermischen von pharmazeutischen Zubereitungen in Form von Emulsionen, in denen eine ersten flüssige Phase in der zweiten flüssigen Phase dispergiert ist.

Dementsprechend betrifft die vorliegende Erfindung auch Vorrichtungen zum Durchmischen von pharmazeutischen Zubereitungen in Form einer Emulsion umfassend eine Kammer, enthaltend
a) eine pharmazeutische Zubereitung umfassend
   i. eine oder mehrere pharmazeutisch wirksame Substanzen und eine erste flüssige Phase,
   ii. eine zweite flüssige Phase, und
b) ein flüssiges Fluorcarbon.

Eine weitere Ausführungsform der vorliegenden Erfindung betrifft die Verwendung von flüssigen Fluorcarbonen zum Vermischen von pharmazeutischen Zubereitungen in Form von Flüssigkeiten, die Flüssigkristallstrukturen bilden.

Dementsprechend betrifft die vorliegende Erfindung auch Vorrichtungen zum Durchmischen von pharmazeutischen Zubereitungen umfassend eine Kammer, enthaltend
a) eine pharmazeutische Zubereitung umfassend eine oder mehrere pharmazeutisch wirksame Substanzen und eine flüssige Phase, die Flüssigkristallstrukturen bildet, und
b) ein flüssiges Fluorcarbon.

Bevorzugte erfindungsgemäße Vorrichtungen zur Injektion von Suspensionen, Emulsionen und Flüssigkeiten, die Flüssigkristallstrukturen sind Autoinjektoren, Fertigspritzen, Pens, oder Injektionspumpen.

Eine weitere Ausführungsform der Erfindung betrifft die Verwendung von flüssigen Fluorcarbonen als Stromstörer zum Vermischen von pharmazeutischen Zubereitungen, in denen eine feste Phase nicht oder nur teilweise in einer flüssigen Phase gelöst ist, wie es bei Pulvern bzw. Lyophilisaten zur Rekonstitution der Fall ist.

In diesem Fall unterstützt der flüssige Stromstörer die Rekonstitution des Pulvers bzw. Lyophilisats, um sicherzustellen, dass die gesamte Menge gelöst wird und sich homogen in der flüssigen Phase verteilt.

Dementsprechend betrifft eine Ausführungsform der vorliegenden Erfindung Vorrichtungen zum Durchmischen von pharmazeutischen Zubereitungen, umfassend eine Kammer, enthaltend
a) eine pharmazeutische Zubereitung umfassend eine flüssige Phase enthaltend einen oder mehrere pharmazeutisch wirksame Stoffe, die in der flüssigen Phase nicht oder nur teilweise gelöst sind, und
b) ein flüssiges Fluorcarbon.

Bevorzugte erfindungsgemäße Vorrichtungen zur Injektion von rekonstituierten Pulvern und Lyophilisaten sind Doppelkammerspritzen.

Die erfindungsgemäßen Vorrichtungen zur Injektion können für die einmalige oder mehrmalige Anwendung geeignet sein. Wegen der einfacheren Handhabung sind Vorrichtungen zur einmaligen Anwendung, d. h. bei der die pharmazeutische Zubereitung als Einmalgabe verabreicht wird, bevorzugt.

Wie bereits erläutert, kann das Vorhandensein einer Gasblase in pharmazeutischen Zubereitungen aus mehreren Gründen nachteilig sein. Durch die Verwendung von erfindungsgemäßen flüssigen Stromstörern wie Fluorcarbonen kann auf den Einsatz gasförmiger Stromstörer verzichtet werden. Bevorzugte Ausführungsformen der Erfindung betreffen daher Verfahren zum Vermischen von flüssigen oder halbfesten pharmazeutischen Zubereitungen, bei denen kein gasförmiger Stromstörer verwendet wird.

Dementsprechend betreffen bevorzugte Ausführungsformen der Erfindung Vorrichtungen, deren die pharmazeutische Zubereitung enthaltende Kammer keinen gasförmigen Stromstörer enthält.

Kleinere Gasblasen können aus abfülltechnischen Gründen oft nicht komplett vermieden werden, tragen aber zur Durchmischung von pharmazeutischen Zubereitungen nicht nennenswert bei. Im Rahmen der vorliegenden Erfindung gelten daher auch Gasphasen als nicht wesentlich, deren Volumen nicht mehr als 5 % des Volumens der Kammer ausfüllt.

Bevorzugte Ausführungsformen der Erfindung betreffen Verfahren zum Vermischen von flüssigen oder halbfesten pharmazeutischen Zubereitungen, bei denen kein fester Stromstörer verwendet wird. Weitere bevorzugte Ausführungsformen der Erfindung sind daher auf Vorrichtungen gerichtet, deren Kammer keine feste Phase enthält, insofern diese nicht Teil der pharmazeutischen Zubereitung (z. B. einer Suspension) ist.

Die Eignung von flüssigen Fluorcarbonen zum Einsatz als Stromstörer in flüssigen oder halbfesten pharmazeutischen Zubereitungen und damit zu deren Durchmischung wird anhand der folgenden Beispiele veranschaulicht.

### Beispiel 1 - Herstellung von PLGA-Partikeln

Partikel aus Poly(milchsäure-co-glykolsäure) (PLGA) wurden mit einer Emulsion-Solvent-Diffusion-Methode hergestellt. Zum Erzeugen der Emulsion wurde ein T 25 digital ULTRA-TURRAX^{®} mit S 25 N - 8 G-Werkzeug (IKA^{®}-Werke GmbH & Co. KG (Staufen, Deutschland)) verwendet.

50 mg PLGA (Resomer RG 503H, Dichte von 1,5 ± 0,1 g/cm³) wurden in 1,5 ml Ethylacetat gelöst. Dann wurden 2,5 ml einer wässrigen Polyvinylalkohol (PVA)-Lösung (2 %) zugegeben. Das resultierende Gesamtvolumen von 4 ml wurde 30 Sekunden bei 10.000 U/min homogenisiert, um eine Ethylacetat-in-Wasser-Emulsion zu erhalten. Um die PLGA-Partikel aus der Emulsion auszufällen, wurden 16 ml der PVA-Lösung zugegeben. Es wurden 5 mal PLGA Partikel nach diesem Verfahren hergestellt. Die Partikel wurden nach dem Abdampfen von Ethylacetat gepoolt und das Volumen mit MilliQ-Wasser auf 200 ml eingestellt, um die endgültige PLGA-Partikelsuspension zu erhalten.

Die Partikelgrößenverteilung (PSD) der resultierenden Partikel wurde mit einem Zetasizer Ultra der Firma Malvern Panalytical GmbH (Kassel, Deutschland) gemessen. Die Partikel hatten eine Z-Average von 1271,8 nm.

### Beispiel 2 - Zentrifugierungsexperiment

Eppendorf-Röhrchen (6 Proben und 6 Kontrollen) wurden mit jeweils 1,5 ml einer nach Beispiel 1 hergestellten PLGA-Partikelsuspension gefüllt. Jede Probe wurden zusätzlich mit 0,25 ml Perfluordecalin (PFD) mit einer Dichte von 1,92 g/cm³ gefüllt.

Alle Röhrchen wurden 30 Minuten lang bei 20 °C zentrifugiert (30.000 g).

Nach der Zentrifugierung wurden Proberöhrchen und Kontrollröhrchen einmal invertiert.

Für eine schematische Darstellung des Zentrifugierungsexperiments siehe Schema 1:

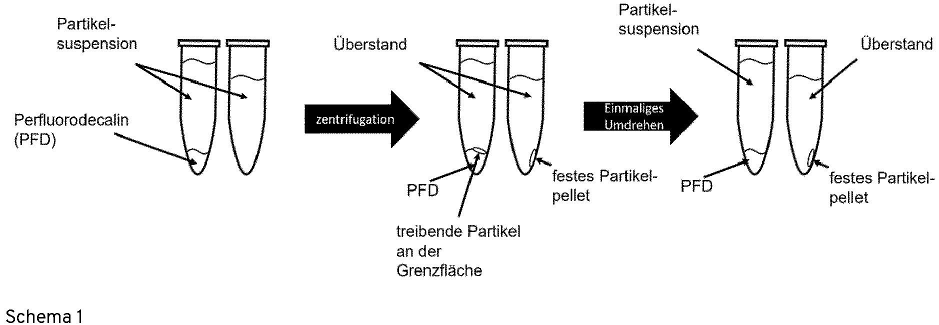

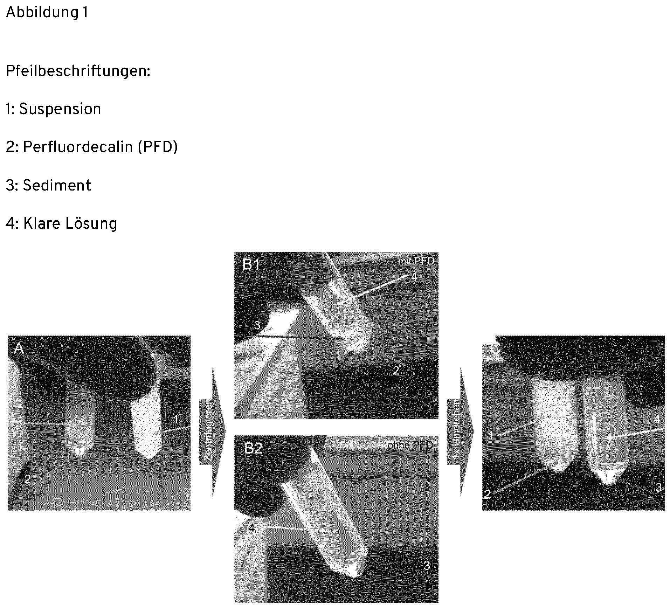

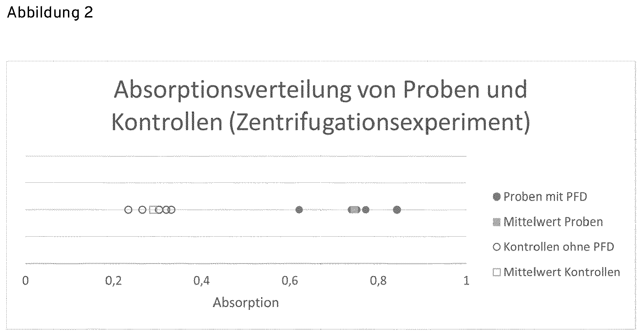

Die Zentrifugierung der Kontrollproben führt zur Bildung eines festen PLGA-Partikelpellets am Boden des Röhrchens. Folglich kam es nach dem Umdrehen der Röhrchen nicht zu einer Resuspendierung und die Flüssigkeit in den Röhrchen der Kontrollproben blieb klar, wie in Abb. 1 C (rechts) zu sehen.

Bei den Proben mit PFD sedimentieren die PLGA-Partikel an der Grenzfläche zwischen der wässrigen Phase und dem PFD, aufgrund einer höheren PFD-Dichte im Vergleich zu PLGA. Dadurch findet keine Sedimentverfestigung statt, und eine erneute Suspension kann leicht durch eine einfache Inversion des Röhrchens erreicht werden, was zu einer Trübung führt, wie in Abb. 1 C (links) zu sehen ist. Diese Ergebnisse werden durch die Messung der Absorptionswerte von Proben mit PFD und Kontrollen ohne PFD bestätigt, wie in Tabelle 1 dargestellt:

**Tabelle 1: Ergebnisse der Absorptionsmessungen**

| | #1 | #2 | #3 | #4 | #5 | #6 | Mittel | S-Abw. |
|---|---|---|---|---|---|---|---|---|
| Proben mit PFD | 0,842 | 0,751 | 0,771 | 0,738 | 0,620 | 0,746 | 0,745 | 0,072 |
| Kontrollen ohne PFD | - | 0,331 | 0,233 | 0,265 | 0,303 | 0,319 | 0,290 | 0,040 |

Tabelle 1 zeigt Absorptionen im Bereich von 0,620 bis 0,842 (Mittelwert 0,745 ± 0,072) für Proben mit PFD und von 0,233 bis 0,331 (Mittelwert 0,290 ± 0,040) für Proben ohne PFD und damit einen signifikanten Unterschied.

Die Ergebnisse zeigen somit deutlich, dass PFD die Verfestigung von sedimentierten Suspensionen verhindert und somit eine vereinfachte Redispergierung ermöglicht.

### Beispiel 3 - Sedimentationsexperiment

Probenkarpulen aus silikonisiertem Glas (3 ml ISO) wurden mit 0,5 ml PFD befüllt und anschließend vollständig mit einer nach Beispiel 1 hergestellten PLGA-Partikelsuspension befüllt. Kontrollkarpulen wurden mit 0,5 ml reinem Wasser befüllt und anschließend vollständig mit Partikelsuspension gefüllt. Auf diese Weise enthielten alle Proben und Kontrollen die gleiche Menge an Partikelsuspension. Alle Karpulen wurden für die Sedimentation liegend gelagert.

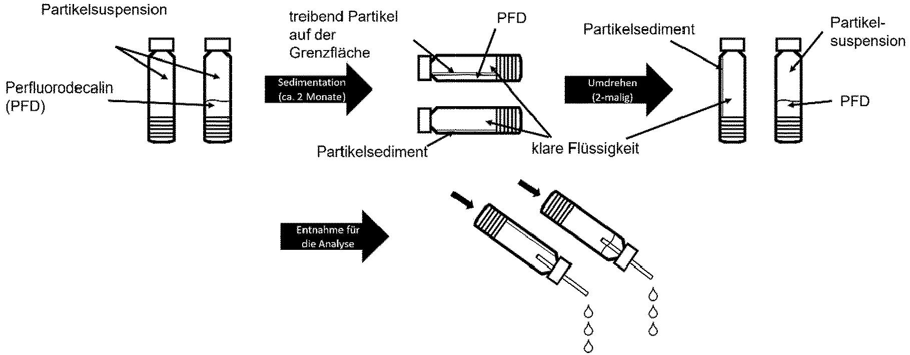

Nach ca. 2 Monaten wurden die Proben und Kontrollen zweimal invertiert, dann wurden ca. 1,5 ml der Flüssigkeit entnommen und photometrisch vermessen. Die Ergebnisse sind in Tabelle 2 dargestellt.

**Tabelle 2: Ergebnisse der Absorptionsmessungen**

| | #1 | #2 | #3 | #4 | #5 | #6 | Mittel | S-Abw. |
|---|---|---|---|---|---|---|---|---|
| Proben mit PFD | 0,927 | 0,455 | 0,604 | 0,733 | 0,842 | 0,687 | 0,708 | 0,168 |
| Kontrolle ohne PFD | 0,014 | - | 0,007 | 0,016 | 0,002 | 0,009 | 0,01 | 0,006 |

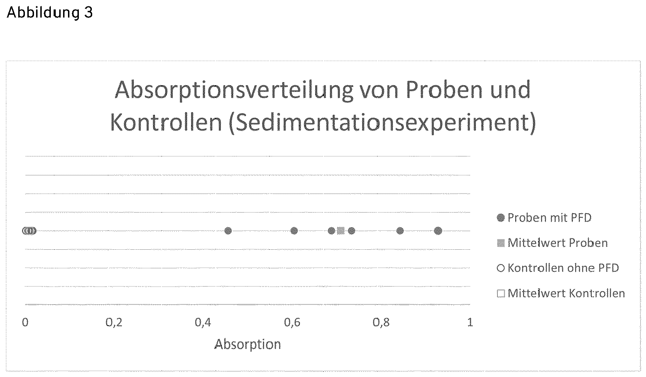

Tabelle 2 zeigt Absorptionen im Bereich von 0,455 bis 0,927 (Mittelwert 0,708 ± 0,168) für Proben mit PFD und von 0,002 bis 0,016 (Mittelwert 0,010 ± 0,006) für Proben ohne PFD und damit einen signifikanten Unterschied, siehe auch Grafik in Abb. 3. Kontrollen und Proben sind signifikant unterschiedlich nach einem *Welch*-test (einseitiger Zweistichproben *Welch*-Test mit 99% Konfidenz und p = 7,83·10⁻⁵). Die Ergebnisse zeigen somit deutlich, dass PFD für die Redispergierung sedimentierter pharmazeutischer Suspensionen eingesetzt werden kann.

## Patentansprüche

1. Verwendung von flüssigen Fluorcarbonen als Stromstörer in flüssigen oder halbfesten pharmazeutischen Zubereitungen, die einen pharmazeutischen Wirkstoff und eine flüssige Phase umfassen.

2. Verwendung von flüssigen Fluorcarbonen zum Vermischen von flüssigen oder halbfesten pharmazeutischen Zubereitungen, die einen pharmazeutischen Wirkstoff und eine flüssige Phase umfassen.

3. Verwendung von flüssigen Fluorcarbonen gemäß Anspruch 1 oder 2, wobei das flüssige Fluorcarbon mindestens 2 und höchstens 18 Kohlenstoffatome, bevorzugt mindestens 3 und höchstens 14 Kohlenstoffatome aufweist.

4. Verwendung von flüssigen Fluorcarbonen gemäß einem der vorhergehenden Ansprüche, wobei das flüssige Fluorcarbon ausgewählt ist aus der Gruppe bestehend aus Perfluordecalin (PFD), Perfluormethyldecalin (PFMD), Perfluor(tert-Butylcyclohexan), Perfluorisopropyldecalin, Perfluorpropan, Perfluorobutan, Perfluorpentan, Perfluorhexan, Perfluorheptan, Perfluoroktan, Perfluornonan, Perfluordekan, Perfluorundekan und Perfluordodekan.

5. Verwendung von flüssigen Fluorcarbonen gemäß einem der vorhergehenden Ansprüche, wobei der pharmazeutische Wirkstoff ausgewählt ist aus der Gruppe bestehend aus Aminosäuren, Peptiden sowie Proteinen.

6. Verwendung von flüssigen Fluorcarbonen gemäß einem der vorhergehenden Ansprüche, wobei die flüssige oder halbfeste pharmazeutische Zubereitung in Form einer Suspension vorliegt.

7. Verwendung von flüssigen Fluorcarbonen gemäß einem der vorhergehenden Ansprüche in einer Vorrichtung zur Injektion von flüssigen oder halbfesten pharmazeutischen Zubereitungen, die eine Kammer umfasst, welche eine pharmazeutische Zubereitung mit einem oder mehreren pharmazeutischen Wirkstoffen und eine flüssige Phase, aber keinen gasförmigen Stromstörer, enthält.

8. Vorrichtung zur Injektion von flüssigen oder halbfesten pharmazeutischen Zubereitungen, umfassend eine Kammer, enthaltend
a) eine pharmazeutische Zubereitung umfassend einen oder mehrere pharmazeutische Wirkstoffe sowie eine flüssige Phase, und
b) ein flüssiges Fluorcarbon.

9. Vorrichtung gemäß Anspruch 8, wobei das flüssige Fluorcarbon mindestens 2 und höchstens 18 Kohlenstoffatome, bevorzugt mindestens 3 und höchstens 14 Kohlenstoffatome aufweist.

10. Vorrichtung gemäß Anspruch 8 oder 9, wobei das flüssige Fluorcarbon ausgewählt ist aus der Gruppe bestehend aus Perfluordecalin (PFD), Perfluormethyldecalin (PFMD), Perfluor(tert-Butylcyclohexan), Perfluorisopropyldecalin, Perfluorpropan, Perfluorobutan, Perfluorpentan, Perfluorhexan, Perfluorheptan, Perfluoroktan, Perfluornonan, Perfluordekan, Perfluorundekan und Perfluordodekan.

11. Vorrichtung nach einem der Ansprüche 8-10, wobei der pharmazeutische Wirkstoff ausgewählt ist aus der Gruppe bestehend aus Aminosäuren, Peptiden, sowie Proteinen.

12. Vorrichtung nach einem der Ansprüche 8-11, wobei die pharmazeutische Zubereitung in Form einer Suspension vorliegt.

13. Vorrichtung nach einem der Ansprüche 8-12, wobei die Vorrichtung zur Injektion ausgewählt ist aus der Gruppe bestehend aus Autoinjektoren, Fertigspritzen, Pens und Injektionspumpen.

14. Vorrichtung nach einem der Ansprüche 8-13, wobei die Kammer zur einmaligen Anwendung vorgesehen ist.

15. Vorrichtung nach einem der Ansprüche 8-14, wobei die Kammer im Wesentlichen keine gasförmige Phase enthält.
